# EUROPÄISCHE PATENTSCHRIFT

(11) **EP 4 340 968 B1**
(45) Veröffentlichungstag und Bekanntmachung des Hinweises auf die Patenterteilung: **16.07.2025**
(21) Anmeldenummer: 22729539.1
(22) Anmeldetag: 16.05.2022
(51) Int. Cl.: B01D 1/28, B01D 3/14, B01D 53/14, C07D 207/26

(54) **ANLAGE UND VERFAHREN ZUM AUFREINIGEN VON RÜCKGEWONNENEM NMP**
SYSTEM AND METHOD FOR PURIFYING RECOVERED NMP
INSTALLATION ET PROCÉDÉ DE PURIFICATION DU NMP RÉCUPÉRÉ

(30) Priorität: 21.05.2021 EP 21175251
(43) Veröffentlichungstag der Anmeldung: 27.03.2024
(73) Patentinhaber: GEA Wiegand GmbH, 76275 Ettlingen (DE)
(72) Erfinder: BECKER, Jörg, 76135 Karlsruhe (DE); LEIBIG, Ralf, 76707 Hambrücken (DE)
(74) Vertreter: Weickmann & Weickmann PartmbB
(86) Internationale Anmeldenummer: PCT/EP2022/063174
(87) Internationale Veröffentlichungsnummer: WO 2022/243238

(56) Entgegenhaltungen:
- CN-A- 108 654 130
- US-A1- 2009 326 307
- US-A1- 2012 241 307
- US-A1- 2013 267 751
- US-A1- 2015 052 940
- US-A1- 2018 185 766
- US-A1- 2020 179 820

## Beschreibung

Die vorliegende Erfindung betrifft eine Anlage zum Aufreinigen von rückgewonnenem NMP aus einer Lithium-Ionen-Batterieproduktion sowie ein Verfahren zum Aufreinigen von rückgewonnenem NMP mittels einer derartigen Anlage.

Insbesondere bei der Herstellung von Elektroden für Lithium-Ionen-Batterien wird als Lösungsmittel NMP (N-Methyl-2-pyrrolidon) in einer besonders hohen Qualität benötigt. Das verwendete NMP wird anschließend in einer Trocknungsanlage aus den Elektrodenmaterialien ausgedampft und nachfolgend kondensiert, um für eine Wiederverwendung rückgewonnen zu werden.

Verfahrensbedingt enthält das rückgewonnene kondensierte NMP, das bei Normalbedingungen einen Siedepunkt von etwa 200°C aufweist, einerseits leicht siedende Verunreinigungen, beispielsweise Wasser, und andererseits hoch siedende Verunreinigungen, beispielsweise Rückstände von Elektrodenmaterial.

Es sind unterschiedliche Verfahren zur Aufreinigung des rückgewonnenen NMP bekannt, wobei beispielsweise auf die JP 2009 212 426 A verwiesen sein soll, in welcher in einem zweistufigen Prozess zunächst einmal leicht siedende Verunreinigungen in einer ersten Kolonne abgetrennt werden, woraufhin in einer zweiten Kolonne das vorgereinigte NMP dann selbst von den hoch siedenden Verunreinigungen abgetrennt wird. In dem genannten Beispiel wird die zur Aufreinigung notwendige Energie in beiden Kolonnen durch thermische Energie, beispielsweise Dampf oder Wärmeträgerflüssigkeit, eingebracht.

Weiterhin sei auf die US 2018/185766 A1, die US 2015/052940 A1, die US 2020/179820 A1, die US 2009/326307 A1 und die US 2013/267751 A1 verwiesen, welche jeweils gattungsgemäße Anlagen gemäß dem Oberbegriff des beiliegenden Anspruchs 1 lehren.

Hierbei zeigt es sich jedoch, dass durch diese Art der Energieeingabe ein hoher Energieverbrauch und hohe laufende Kosten beim Betrieb der entsprechenden Anlage entstehen. Es besteht demzufolge Verbesserungspotential hinsichtlich der Energieeffizienz sowie der Betriebskosten von aus dem Stand der Technik bekannten Anlagen und Verfahren zum Aufreinigen von rückgewonnenem NMP aus einer Lithium-Ionen-Batterieproduktion.

Die Möglichkeit, thermische Energie durch den Einsatz von Wärmepumpentechnologie zu reduzieren, wird jedoch zunächst einmal durch die sehr hohe Temperaturdifferenz von über 80 Kelvin zwischen der Kondensationstemperatur des Kopfprodukts (insbesondere der Siedetemperatur von Wasser) und der Verdampfungstemperatur des NMP im Sumpf der Kolonne erschwert.

Hierbei ist andererseits zu beachten, dass verfahrensbedingt das NMP bei seiner Rückgewinnung typischerweise mit weniger als 5 % Wasser- bzw. Leichtsiederanteil anfällt, wodurch während der Aufreinigung des NMP nur ein geringer Energieanteil zur Abreicherung der Leichtsieder aufgewendet werden muss, während der wesentliche Anteil der Energie zum Verdampfen des NMP bzw. zur Schwersiederabtrennung in einem zweiten Prozessschritt benötigt wird.

Demzufolge ist es die Aufgabe der vorliegenden Erfindung, bekannte Anlagen und Verfahren zum Aufreinigen von rückgewonnenem NMP in einer Weise weiterzubilden, die eine erhöhte Energieeffizienz sowie einen kostengünstigeren Betrieb ermöglicht.

Zu diesem Zweck umfasst eine erfindungsgemäße Anlage zum Aufreinigen von rückgewonnenem NMP aus einer Lithium-Ionen-Batterieproduktion eine erste Kolonne zum Abtrennen von leichtsiedenden Verunreinigungen, welche in ihrem mittleren Teil eine Zuführung für das rückgewonnene NMP aufweist und welche mittels eines ersten Verdampfers durch Zufuhr von thermischer Energie beheizbar ist, sowie eine zweite Kolonne zum Abtrennen von hochsiedenden Verunreinigungen, wobei eine Verbindungsleitung von einem Unterteil der ersten Kolonne zu einem Unterteil der zweiten Kolonne vorgesehen ist. Erfindungsgemäß ist hierbei die zweite Kolonne mittels eines zweiten Verdampfers mit einem Eingang und einem Ausgang für gereinigtes NMP durch Kondensation der gereinigten NMP-Brüden beheizbar und der zweiten Kolonne ist ein Verdichter-Abschnitt zugeordnet, welcher von einem Kopf der zweiten Kolonne zu dem Eingang des zweiten Verdampfers verläuft und einen mechanischen Brüdenverdichter zur Druckerhöhung und somit zur Erhöhung der Kondensationstemperatur der gereinigten NMP-Brüden der zweiten Kolonne umfasst.

Demzufolge beruht die Funktionsweise der erfindungsgemäßen Anlage darauf, einen Teil der zur Reinigung des zugeführten NMP notwendigen Energie mittels mechanischer Brüdenverdichtung nach dem Wärmepumpenprinzip einzubringen. Hierbei ist zum Erreichen der erforderlichen Produktreinheit eine Anordnung gewählt worden, bei der die beiden Trennaufgaben, nämlich Leichtsiederabtrennung und Hochsiederabtrennung, auf zwei Kolonnen aufgeteilt sind. Hieraus ergibt sich für die zweite Trennaufgabe, also der Hochsiederabtrennung, eine deutlich reduzierte Temperaturdifferenz zwischen der Kondensationstemperatur des Kopfprodukts, nämlich des aufgereinigten NMP, und der Verdampfungstemperatur des mit Hochsiedern verunreinigten NMP im Sumpf der Kolonne.

Auf diese Weise eignet sich für jene zweite Trennaufgabe insbesondere der Einsatz einer mechanischen Brüdenverdichtung, und mit einer Leistungszahl, definiert als thermische Heizleistung geteilt durch elektrische Leistung der mechanischen Brüdenverdichtung, von etwa 20 im praktischen Betrieb kann bei dieser Trennaufgabe eine deutlich erhöhte Energieeffizienz gegenüber den oben beschriebenen bekannten Verfahren erreicht werden.

Hierbei spielt eine wesentliche Rolle, dass für diese zweite Trennaufgabe das komplette NMP inklusive des erforderlichen Rücklaufs verdampft werden muss und hierfür mehr als 80 % der Gesamtenergie des Betriebs der kompletten erfindungsgemäßen Anlage aufgewendet werden müssen. Somit kann angesichts der oben genannten praktisch erreichbaren Leistungszahl durch den Einsatz der mechanischen Brüdenverdichtung eine Energieeinsparung der Gesamtanlage von bis zu 75 % erzielt werden, wodurch die Installation und der Betrieb des mechanischen Brüdenverdichters bereits in kurzer Zeit amortisiert werden können.

Um die Effizienz der erfindungsgemäßen Anlage weiter zu erhöhen, kann der Zuführung der ersten Kolonne ein Wärmetauscher, insbesondere ein Plattenwärmetauscher, vorgelagert sein, welcher andererseits mit dem Ausgang des zweiten Verdampfers gekoppelt ist. Hierdurch kann das aus der Anlage entnommene rückgewonnene NMP einen weiteren Teil seiner während des Prozesses aufgenommenen Wärme an das zuzuführende rückzugewinnende NMP abgeben, wodurch die entsprechende Energie in der Anlage verbleibt und dementsprechend nicht nachgeliefert werden muss oder verlorengeht.

Die erfindungsgemäße Anlage umfasst ferner einen Kondensator zum Kondensieren der leichtsiedenden Verunreinigung, welcher mit dem Kopf der ersten Kolonne gekoppelt ist. Das derart erzeugte Kondensat kann einerseits aus der Anlage als Abfallprodukt entnommen werden und andererseits auch teilweise als Rücklauf in die erste Kolonne zurückgeführt werden.

Da dieser Kondensator denjenigen Punkt in der Anlage darstellt, an welchem der geringste Druck herrscht, kann ihm eine ferner eine Vakuumpumpe zugeordnet sein, welche dazu eingerichtet ist, in der Anlage einen gegenüber der Umgebung verminderten Druck aufrechtzuerhalten und einzustellen. Da in derart komplexen Anlagen stets damit zu rechnen ist, dass über minimale Undichtigkeiten Umgebungsluft an verschiedenen Stellen eindringen kann, wird diese Vakuumpumpe benötigt, um ein Senkung der Betriebstemperatur und damit eine weitere Energieeinsparung verglichen mit Normalbedingungen zu erreichen.

Besondere Eignung für die Verwendung als der erste und/oder der zweite Verdampfer weisen unterschiedliche Bauformen von Fallstromverdampfern oder Umlaufverdampfern auf, während jedoch prinzipiell auch der Einsatz anderer Verdampfertypen an dieser Stelle zur Eingabe der für die entsprechenden Prozesse notwendigen Wärme in die Anlage denkbar ist.

In der erfindungsgemäßen Anlage ist zwischen dem Unterteil der ersten Kolonne und dem Unterteil der zweiten Kolonne eine Gaspendelleitung vorgesehen und zwischen dem zweiten Verdampfer und einem mittleren Abschnitt der ersten Kolonne eine Entlüftungsleitung mit einem Regelventil vorgesehen. Durch das Bereitstellen der Gaspendelleitung wird ein Druck- und Temperaturausgleich zwischen den Unterteilen der ersten und zweiten Kolonne erzielt, während durch die Entlüftungsleitung ein geeignetes Druckgefälle an den entsprechenden Punkten der Anlage eingestellt werden kann.

Das Vorsehen dieser zusätzlichen Komponenten in der erfindungsgemäßen Anlage ist in mehrerlei Hinsicht vorteilhaft. Zum einen kann eine Einkopplung von thermischer Überschussenergie des mittels des mechanischen Brüdenverdichters beheizten zweiten Verdampfers in die thermisch beheizte erste Kolonne stattfinden, wodurch ggf. die Notwendigkeit einer zusätzlichen Vorrichtung, wie insbesondere eines dem zweiten Verdampfer nachgeschalteten zusätzlichen Kondensators oder eines Kühlers entfallen kann. Zum zweiten kann auf eine Entlüftung von Inertgasen über nachgeschaltete Systeme verzichtet werden, welche ebenfalls zusätzliche Vorrichtungen erfordern würde und die Gefahr einer Verschleppung von Spuren nicht abgetrennter Leichtsieder in das Produkt durch vollständige Kondensation mit sich bringt, indem eine partielle und über die Entlüftungsleitung geregelte Kondensation in dem zweiten Verdampfer durchgeführt wird und der Entlüftungsbrüden dieses zweiten Verdampfers auf diesem Weg in die erste Kolonne eingekoppelt wird. Zuletzt kann durch das Vorsehen der Gaspendelleitung insbesondere während eines Anfahrens der Anlage die Beheizungsvorrichtung der ersten Kolonne zum Durchheizen des zweiten Verdampfers verwendet werden, da zu diesem Zeitpunkt durch den mechanischen Brüdenverdichter noch keine Energie in die Anlage eingegeben werden kann. Somit entfällt auch hier die Notwendigkeit einer weiteren Vorrichtung, insbesondere eines thermisch beheizten Vorwärmers des zweiten Verdampfers.

Weiterhin kann in der erfindungsgemäßen Anlage die erste Kolonne einen Tassenabzug umfassen, von welchem aus die Verbindungsleitung zu dem Unterteil der zweiten Kolonne verläuft. Hierdurch wird der Ablauf der ersten Kolonne bereits vor seinem Eintritt in deren Kolonnensumpf in die mittels mechanischer Brüdenverdichtung beheizte zweite Kolonne ausgeleitet. Somit kann in der ersten Kolonne das NMP unter einer nur unwesentlichen Konzentrationserhöhung der hochsiedenden Verunreinigungen von den leichtsiedenden Verunreinigungen gereinigt werden.

In diesem Zusammenhang kann die Anlage ferner eine Flüssigkeitsrückführung umfassen, welche dazu eingerichtet ist, aus dem Sumpf der zweiten Kolonne entnommene hochsiedende Verunreinigungen wenigstens teilweise in den Unterteil der ersten Kolonne einzugeben. Demzufolge können die hochsiedenden Verunreinigungen in dem Sumpf der ersten Kolonne unter Zuführung thermischer Energie aufkonzentriert werden, was es ermöglicht, den durch den mechanischen Brüdenverdichter beheizten Teil der Anlage von schädlichen Einflüssen bedingt durch hohe Schwersiederkonzentrationen freizuhalten, um mit möglichst geringem apparativen Aufwand den Produktverlust durch eine Aufkonzentrierung der hochsiedenden Verunreinigungen zu minimieren. Hierbei eignet sich eine derartige Ausgestaltung insbesondere für hochsiedende Verunreinigungen mit geringem Dampfdruck, also beispielsweise Rückstände von Elektrodenmaterial.

Ferner wird durch die genannten Maßnahmen erreicht, dass durch eine verminderte Aufkonzentrierung der hochsiedenden Verunreinigungen in dem durch den mechanischen Brüdenverdichter beheizten Anlagenteil eine Siedepunkterhöhung und ein damit verbundener erhöhter Strombedarf des Brüdenverdichters entfällt. Zudem kann ebenfalls auf einen dem Brüdenverdichter zugeordneten Verdampfer verzichtet werden, welcher ggf. aufgrund von potenziell ausfällenden Feststoffen, einer erhöhten Viskosität und einem verschlechterten Wärmeübergang an dieser Stelle notwendig sein könnte.

Um die hochsiedenden Verunreinigungen in derartigen Ausführungsformen aus der Anlage zu entfernen, kann diese ferner eine Austragleitung zum Ausleiten von hochsiedenden Verunreinigungen aus dem Unterteil der ersten Kolonne zu einer Entnahmeleitung umfassen.

Wie bereits angedeutet, betrifft die vorliegende Erfindung gemäß einem zweiten Aspekt ein Verfahren zum Aufreinigen von rückgewonnenem NMP aus einer Lithium-Ionen-Batterieproduktion mittels einer erfindungsgemäßen Anlage der eben beschriebenen Art, umfassend die Schritte:
- Eingeben des rückgewonnenen NMP in den mittleren Teil der ersten Kolonne;
- Beheizen der ersten Kolonne mittels des ersten Verdampfers durch Zufuhr von thermischer Energie;
- Entnehmen von leichtsiedenden Verunreinigungen aus dem Kopf der ersten Kolonne;
- Überführen von vorgereinigtem NMP aus dem Unterteil oder dem Tassenabzug der ersten Kolonne in das Unterteil der zweiten Kolonne;
- Verdampfen des vorgereinigten NMPs in dem zweiten Verdampfer und Leiten davon durch die zweite Kolonne;
- Entnehmen des dampfförmigen NMP an dem Kopf der zweiten Kolonne und Verdichten davon in dem Verdichter-Abschnitt mittels des mechanischen Brüdenverdichters;
- Eingeben des verdichtenden NMP in den Eingang des zweiten Verdampfers, wobei das NMP in dem zweiten Verdampfer kondensiert;
- Entnehmen des kondensierten NMP an dem Ausgang des zweiten Verdampfers; und
- Entnehmen von hochsiedenden Verunreinigungen aus dem Sumpf der zweiten Kolonne.

Hierbei ist aus den oben angeführten Gründen mit dem erfindungsgemäßen Verfahren eine erhebliche Einsparung an Energie- und Betriebskosten im Betrieb der Anlage zu erzielen.

Weiterhin kann zur Senkung der Betriebstemperatur und damit zur weiteren Energieeinsparung in der ersten und der zweiten Kolonne ein gegenüber Normalbedingungen verminderter Druck aufrechterhalten werden, insbesondere von weniger als 100 mbar Absolutdruck.

Wenngleich die Art und Weise des Zuführens von thermischer Energie zu dem ersten Verdampfer im Rahmen der vorliegenden Erfindung mittels beliebiger Techniken bewerkstelligt werden kann, so kann dies insbesondere mittels Dampf oder einer Wärmeträgerflüssigkeit erfolgen, um die bereits angesprochene hohe Heiztemperatur zum Betrieb der ersten Kolonne erzielen zu können.

Weiterhin kann im Betrieb der erfindungsgemäßen Anlage gemäß dem hier vorgestellten Verfahren ein Teil des kondensierten NMP aus dem zweiten Verdampfer als Rücklauf in die zweite Kolonne zurückgeführt werden.

Wie bereits weiter oben angedeutet, können ferner die dem Kopf der ersten Kolonne entnommenen leichtsiedenden Verunreinigungen in dem Kondensator kondensiert und vorzugsweise teilweise als Rücklauf in die erste Kolonne rückgeführt werden.

Während verfahrensbedingt das rückgewonnene NMP typischerweise weniger als 5 % an leichtsiedenden Verunreinigungen enthalten kann, so können ferner in der ersten Kolonne die leicht siedenden Verunreinigungen in dem vorgereinigten NMP auf weniger als 0,1 %, vorzugsweise auf weniger als 0,05%, abgereichert werden, um die erforderliche Produktqualität für das letztlich aus der Anlage entnommene gereinigte NMP erzielen zu können.

In der oben bereits angesprochenen Ausführungsform einer erfindungsgemäßen Anlage mit einer Flüssigkeitsrückführung vom Sumpf der zweiten Kolonne zu dem Unterteil der ersten Kolonne können dementsprechend ferner die aus dem Sumpf der zweiten Kolonne entnommenen hochsiedenden Verunreinigungen wenigstens teilweise in den Unterteil der ersten Kolonne eingegeben werden, um die oben ebenfalls angesprochenen Vorteile im Betrieb der Anlage zu erzielen.

Insbesondere kann in solchen Ausführungsformen die Konzentration der hochsiedenden Verunreinigungen in dem Sumpf der zweiten Kolonne während eines Betriebs der Anlage bezogen auf die anfängliche Konzentration in dem rückgewonnenen NMP etwa 1 bis 5 betragen, und die Konzentration der hochsiedenden Verunreinigungen in dem Unterteil der der ersten Kolonne gegenüber diesem Wert erhöht sein und im Bereich von größer 1 bis etwa 100 liegen. In einem energetisch besonders vorteilhaften Prozess liegen hierbei die beiden genannten Werte bei etwa 3 bzw. im Bereich von 50 bis 60.

Weitere Merkmale und Vorteile der vorliegenden Erfindung werden aus der nachfolgenden Beschreibung einer Ausführungsform davon noch deutlicher, wenn diese zusammen mit den beiliegenden Figuren betrachtet wird. Diese zeigen im Einzelnen:
- Figur 1: eine schematische Darstellung einer erfindungsgemäßen Anlage zum Aufreinigen von rückgewonnenem NMP; und
- Figur 2: eine schematische Darstellung einer weiteren Variante einer erfindungsgemäßen Anlage

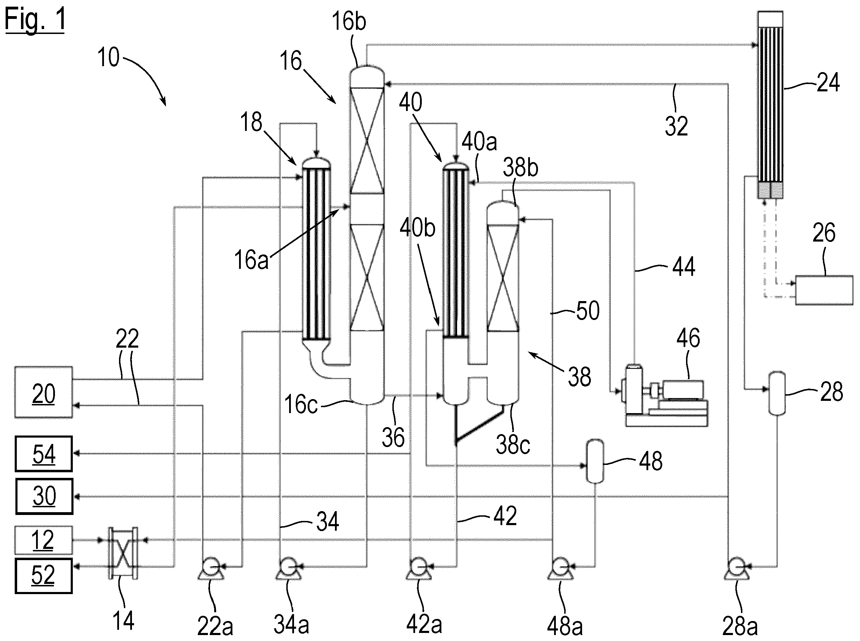

In Figur 1 ist die erfindungsgemäße Anlage zum Aufreinigen von rückgewonnenem NMP ganz allgemein mit dem Bezugszeichen 10 bezeichnet. Hierbei wird zunächst beim Punkt 12 rückgewonnenes NMP aus einer Lithium-Ionen-Batterieproduktion in die Anlage eingegeben, welches typischerweise weniger als 5 % an leichtsiedenden Verunreinigungen, insbesondere Wasser, sowie zusätzlich hochsiedende Verunreinigungen, wie beispielsweise verbleibendes Elektrodenmaterial, enthält.

Das in die Anlage eingegebene NMP durchläuft in der Anlage 10 zunächst einen Wärmetauscher 14, in welchem es vorgewärmt wird, bevor es beim Punkt 16a in den mittleren Teil einer ersten Kolonne 16 eingegeben wird. Hierbei ist der ersten Kolonne 16 ein erster Verdampfer 18 zugeordnet, welchem vom Punkt 20 Dampf oder Wärmeträgerflüssigkeit mittels eines von einer Pumpe 22a angetriebenen Kreislaufs 22 zum Betrieb zugeführt werden kann. Durch die Zuführung von thermischer Energie in die erste Kolonne 16 mittels ersten Verdampfers 18 verdampfen dort die leicht siedenden Verunreinigungen des zugeführten, rückzugewinnenden NMP und können am Kopfbereich 16b der ersten Kolonne entnommen und einem Kondensator 24 zugeführt werden, in welchem mittels einer Wasserkühlung 26 eine Kondensation der leicht siedenden Verunreinigungen erzielt wird. Diese kondensierten Verunreinigungen können anschließend in einem Tank 28 gesammelt und mittels einer Pumpe 28a am Punkt 30 als Abfallprodukt aus der Anlage entnommen sowie teilweise über eine Rückführleitung 32 als Rücklauf in die erste Kolonne 16 zurückgeführt werden.

Demhingegen ist am Unterteil 16c der ersten Kolonne einerseits ein Umwälzkreislauf 34 mit einer Pumpe 34a zum Zuführen von dort entnommenem vorgereinigtem NMP zu dem ersten Verdampfer 18 sowie andererseits eine Verbindungsleitung 36 vorgesehen, über welche das vorgereinigte NMP vom Unterteil 16c der ersten Kolonne 16 in das Unterteil 38c einer zweiten Kolonne 38 überführt werden kann. Die zweite Kolonne 38 ist dazu betreibbar, das bereits vorgereinigte NMP von hoch siedenden Verunreinigungen zu trennen, indem das NMP selbst verdampft und im Kopfbereich 38b dieser zweiten Kolonne 38 entnommen wird.

Hierzu ist dieser zweiten Kolonne 38 ein zweiter Verdampfer 40 zugeordnet, welchem einerseits mittels eines durch eine Pumpe 42a angetriebenen Kreislaufs 42a dem Unterteil 38c der zweiten Kolonne 38 entnommenes, mit hochsiedenden Verunreinigungen angereichertes Sumpfprodukt zugeführt wird, während andererseits zur Beheizung des zweiten Verdampfers 40 der im Kopfbereich 38b der zweiten Kolonne 38 entnommene Brüden, d.h. verdampftes gereinigtes NMP, zugeführt wird. Dieser Brüden wurde zwischenzeitlich in einem von dem Kopfbereich 38b der zweiten Kolonne 38 zu dem entsprechenden Eingang 40a des zweiten Verdampfers 40 verlaufenden Verdichter-Abschnitt 44 mittels eines mechanischen Brüdenverdichters 46 verdichtet und so in seinem Energiegehalt und seiner Kondensationstemperatur erhöht, wodurch in dieser Weise die für den Betrieb der zweiten Kolonne 38 notwendige Energie eingegeben wird.

Bei Durchlauf durch den zweiten Verdampfer 40, welcher beispielsweise als Fallstromverdampfer oder Umlaufverdampfer ausgeführt sein kann, was im Übrigen ebenfalls für den ersten Verdampfer 18 gilt, kondensiert das gereinigte NMP und kann anschließend von einem Ausgang 40b des zweiten Verdampfers 40 in einen Tank 48 überführt werden. Von dort aus kann es anschließend mittels einer Pumpe 48a einerseits über eine Rückführleitung 50 als Rücklauf in die zweite Kolonne 38 zurückgeführt werden und andererseits über eine Ausgangsleitung 52 als Endprodukt aus der Anlage 10 entnommen werden.

Das gereinigte und kondensierte NMP kann abschließend bei einem Durchlauf durch den oben bereits angesprochenen Wärmetauscher 14 weitere Wärme an das am Punkt 12 neu zugeführte, rückzugewinnende NMP abgeben.

In der erfindungsgemäßen Anlage 10 wird erfindungsgemäß durch die Verwendung des mechanischen Brüdenverdichters 46 zur Beheizung des zweiten Verdampfers 40 eine erhebliche Energieeinsparung im Betrieb erzielt, wobei der Vollständigkeit halber zuletzt noch auf die Entnahmeleitung 54 hingewiesen sein soll, mittels welcher die hoch siedenden Verunreinigungen aus dem Unterteil 38c der zweiten Kolonne 38, welche teilweise, wie oben angesprochen, als Rücklauf in den zweiten Verdampfer 40 umgewälzt werden, ebenfalls aus der Anlage 10 als Abfallprodukt entnommen werden können.

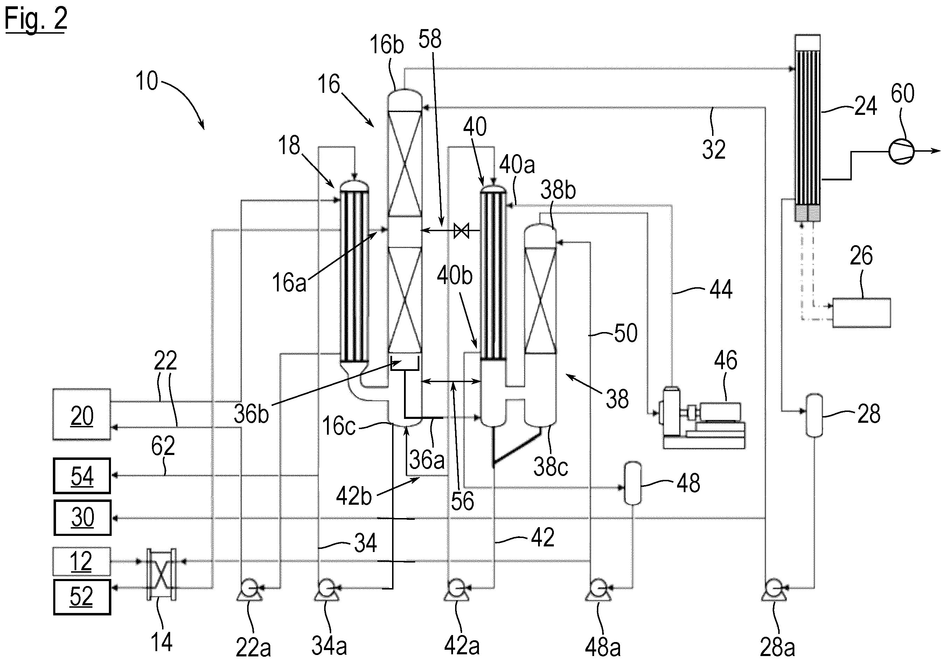

Demhingegen zeigt die Figur 2 eine weitere Variante einer erfindungsgemäßen Anlage, welche sich hinsichtlich einiger vorteilhafter konstruktiver Merkmale von der in Figur 1 gezeigten Ausführungsform unterscheidet, auf welche im Folgenden eingegangen werden wird, während auf eine erneute Beschreibung von identischen Komponenten verzichtet sein soll.

Zum einen umfasst die erste Kolonne 16 in dieser Variante einen Tassenabzug 36b, von welchem aus eine Verbindungsleitung 36a zu dem Unterteil 38c der zweiten Kolonne 38 verläuft. Diese Anordnung ersetzt in dieser Variante die einfache Verbindungsleitung 36 gemäß der ersten Ausführungsform und erstreckt sich im Sinne der vorliegenden Erfindung ebenfalls von dem Unterteil 16c der ersten Kolonne 16 zu dem Unterteil 38c der zweiten Kolonne 38.

Ferner ist eine Flüssigkeitsrückführung 42b vorgesehen, welche dazu eingerichtet ist, aus dem Sumpf 38c der zweiten Kolonne 38 entnommene hochsiedende Verunreinigungen wenigstens teilweise in den Unterteil 16c der ersten Kolonne 16 einzugeben. Da demzufolge in dieser Variante eine Anreicherung der hochsiedenden Verunreinigungen im Unterteil 16c der ersten Kolonne 16 erfolgt, ist ferner eine Austragleitung 62 bereitgestellt, welche ein Ausleiten von hochsiedenden Verunreinigungen aus dem Unterteil 16c der ersten Kolonne 16 zu der Entnahmeleitung 54 ermöglicht.

Weiterhin ist in der in Figur 2 gezeigten Variante mittels einer Gaspendelleitung 56 eine Verbindung zwischen dem Unterteil 16c der ersten Kolonne 16 und dem Unterteil 38c der zweiten Kolonne 38 geschaffen, sowie gleichzeitig zwischen einem oberen Teil des zweiten Verdampfers 40 und einem mittleren Abschnitt der ersten Kolonne 16 eine Entlüftungsleitung mit einem Regelventil 58 bereitgestellt.

Zuletzt sei auch noch auf die Vakuumpumpe 60 hingewiesen, welche dem Kondensator 24 zugeordnet ist und demzufolge an demjenigen Punkt der Anlage 10 gemäß Figur 2, an welchem ein minimaler Druck herrscht, eine Erzeugung und Einstellung eines verminderten Drucks in der Anlage ermöglicht.

## Patentansprüche

1. Anlage (10) zum Aufreinigen von rückgewonnenem NMP aus einer Lithium-Ionen-Batterieproduktion, umfassend:
- eine erste Kolonne (16) zum Abtrennen von leichtsiedenden Verunreinigungen, welche in ihrem mittleren Teil eine Zuführung (16a) für das rückgewonnene NMP aufweist und welche mittels eines ersten Verdampfers (18) durch Zufuhr von thermischer Energie beheizbar ist; und
- eine zweite Kolonne (38) zum Abtrennen von hochsiedenden Verunreinigungen,
- einen Kondensator (24) zum Kondensieren der leichtsiedenden Verunreinigungen, welcher mit einem Kopf (16b) der ersten Kolonne (16) gekoppelt ist
wobei eine Verbindungsleitung (36) von einem Unterteil (16c) der ersten Kolonne (16) zu einem Unterteil (38c) der zweiten Kolonne (38) vorgesehen ist; wobei die zweite Kolonne (38) mittels eines zweiten Verdampfers (40) mit einem Eingang (40a) und einem Ausgang (40b) für gereinigtes NMP beheizbar ist, und
der zweiten Kolonne (38) ein Verdichter-Abschnitt (44) zugeordnet ist, welcher von einem Kopf (38b) der zweiten Kolonne (38) zu dem Eingang (40a) des zweiten Verdampfers (40) verläuft und einen mechanischen Brüdenverdichter (46) zum Eingaben von Energie an den Brüden der zweiten Kolonne (38) umfasst,
**dadurch gekennzeichnet, dass** zwischen dem Unterteil (16c) der ersten Kolonne (16) und dem Unterteil (38c) der zweiten Kolonne (38) eine Gaspendelleitung (56) vorgesehen ist und zwischen dem zweiten Verdampfer (40) und einem mittleren Abschnitt der ersten Kolonne (16) eine Entlüftungsleitung mit einem Regelventil (58) vorgesehen ist.

2. Anlage (10) nach Anspruch 1,
wobei der Zuführung (16a) der ersten Kolonne (16) ein Wärmetauscher (14), insbesondere ein Plattenwärmetauscher, vorgelagert ist, welcher andererseits mit dem Ausgang (40b) des zweiten Verdampfers (40) gekoppelt ist.

3. Anlage nach einem der vorhergehenden Ansprüche,
wobei dem Kondensator (24) eine Vakuumpumpe (60) zugeordnet ist, welche dazu eingerichtet ist, in der Anlage einen verminderten Druck aufrechtzuerhalten.

4. Anlage (10) nach einem der Ansprüche 1 bis 3,
wobei der erste und/oder der zweite Verdampfer (18, 40) als Fallstromverdampfer oder Umlaufverdampfer ausgeführt ist/sind.

5. Anlage nach einem der vorhergehenden Ansprüche,
wobei die erste Kolonne (16) einen Tassenabzug (36b) umfasst, von welchem aus die Verbindungsleitung (36a) zu dem Unterteil (38c) der zweiten Kolonne (38) verläuft.

6. Anlage nach Anspruch 5,
ferner umfassend eine Flüssigkeitsrückführung (42b), welche dazu eingerichtet ist, aus dem Sumpf (38c) der zweiten Kolonne (38) entnommene hochsiedende Verunreinigungen wenigstens teilweise in den Unterteil (16c) der ersten Kolonne (16) einzugeben.

7. Anlage nach Anspruch 6,
ferner umfassend eine Austragleitung (62) zum Ausleiten von hochsiedenden Verunreinigungen aus dem Unterteil (16c) der ersten Kolonne (16) zu einer Entnahmeleitung (54).

8. Verfahren zum Aufreinigen von rückgewonnenem NMP aus einer Lithium-Ionen-Batterieproduktion mittels einer Anlage (10) nach einem der vorhergehenden Ansprüche, umfassend die Schritte:
- Eingeben des rückgewonnenen NMP in den mittleren Teil der ersten Kolonne (16);
- Beheizen der ersten Kolonne (16) mittels des ersten Verdampfers (18) durch Zufuhr von thermischer Energie;
- Entnehmen von leichtsiedenden Verunreinigungen an dem Kopf (16b) der ersten Kolonne (16);
- Überführen von vorgereinigtem NMP aus dem Unterteil (16c) der ersten Kolonne (16) oder dem Tassenabzug (36b) in das Unterteil (38c) der zweiten Kolonne (38);
- Verdampfen des vorgereinigten NMP in dem zweiten Verdampfer (40) und Leiten davon durch die zweite Kolonne (38);
- Entnehmen des dampfförmigen NMP an dem Kopf (38b) der zweiten Kolonne (38) und Verdichten davon in dem Verdichter-Abschnitt (44) mittels des mechanischen Brüdenverdichters (46);
- Eingeben des verdichteten NMP in den Eingang (40a) des zweiten Verdampfers (40), wobei das NMP in dem zweiten Verdampfer (40) kondensiert;
- Entnehmen des kondensierten NMP an dem Ausgang (40b) des zweiten Verdampfers (40); und
- Entnehmen von hochsiedenden Verunreinigungen aus dem Sumpf (38c) der zweiten Kolonne (38).

9. Verfahren nach Anspruch 8,
wobei in der ersten und der zweiten Kolonne (16, 38) ein verminderter Druck aufrecht erhalten wird, insbesondere von weniger als 100 mbar.

10. Verfahren nach einem der Ansprüche 8 und 9,
wobei das Zuführen von thermischer Energie zu dem ersten Verdampfer (18) mittels Dampf oder einer Wärmeträgerflüssigkeit erfolgt.

11. Verfahren nach einem der Ansprüche 8 bis 10,
wobei ein Teil des kondensierten NMP aus dem zweiten Verdampfer (40) als Rücklauf in die zweite Kolonne (38) rückgeführt wird.

12. Verfahren nach einem der Ansprüche 8 bis 11,
wobei die an dem Kopf (16b) der ersten Kolonne (16) entnommenen leichtsiedenden Verunreinigungen in dem Kondensator (24) kondensiert und vorzugsweise teilweise als Rücklauf in die erste Kolonne (16) zurückgeführt werden.

13. Verfahren nach einem der Ansprüche 8 bis 12,
wobei das rückgewonnene NMP weniger als 5 % an leichtsiedenden Verunreinigungen enthält; und/oder
wobei in der ersten Kolonne (16) die leichtsiedenden Verunreinigungen in dem vorgereinigten NMP auf weniger als 0,1 %, vorzugsweise aus weniger als 0,05%, abgereichert werden.

14. Verfahren nach einem der Ansprüche 8 bis 13,
wobei die aus dem Sumpf (38c) der zweiten Kolonne (38) entnommenen hochsiedenden Verunreinigungen wenigstens teilweise in den Unterteil (16c) der ersten Kolonne (16) eingegeben werden.

15. Verfahren nach Anspruch 14,
wobei die Konzentration der hochsiedenden Verunreinigungen in dem Sumpf (38c) der zweiten Kolonne (38) während eines Betriebs der Anlage bezogen auf die anfängliche Konzentration in dem rückgewonnenen NMP etwa 1 - 5 beträgt, und die Konzentration der hochsiedenden Verunreinigungen in dem Unterteil der (16c) der ersten Kolonne (16) gegenüber diesem Wert erhöht ist und im Bereich von größer 1 bis etwa 100 liegt.

## Claims

1. System (10) for the purification of recovered NMP from a lithium-ion battery production, comprising:
- a first column (16) for separating low-boiling impurities, which has a feed (16a) for the recovered NMP in its middle part and can be heated by supplying thermal energy via a first evaporator (18); and
- a second column (38) for separating high-boiling impurities,
- a condenser (24) for condensing the low-boiling impurities, which is coupled to a head (16b) of the first column (16)
wherein a connecting line (36) is provided from a lower part (16c) of the first column (16) to a lower part (38c) of the second column (38); wherein the second column (38) can be heated by means of a second evaporator (40) with an inlet (40a) and an outlet (40b) for purified NMP, and
a compressor section (44) is assigned to the second column (38), which runs from a head (38b) of the second column (38) to the inlet (40a) of the second evaporator (40) and includes a mechanical vapour compressor (46) for inputting energy to the vapour of the second column (38),
**characterised in that** a gas pendulum line (56) is provided between the lower part (16c) of the first column (16) and the lower part (38c) of the second column (38), and a vent line with a control valve (58) is provided between the second evaporator (40) and a middle section of the first column (16).

2. System (10) according to claim 1,
wherein a heat exchanger (14), in particular a plate heat exchanger, is positioned upstream of the feed (16a) of the first column (16), which is otherwise coupled with the outlet (40b) of the second evaporator (40).

3. System according to any one of the preceding claims,
wherein the condenser (24) is associated with a vacuum pump (60) which is configured to maintain a reduced pressure in the system.

4. System (10) according to any one of claims 1 to 3,
wherein the first and/or the second evaporator (18, 40) is/are designed as a falling film evaporator or a circulation evaporator.

5. System according to any one of the preceding claims,
wherein the first column (16) includes a cup draw-off (36b), from which the connecting line (36a) extends to the lower part (38c) of the second column (38).

6. System according to claim 5,
further comprising a fluid return (42b) which is arranged to at least partially introduce high-boiling impurities extracted from the sump (38c) of the second column (38) into the lower part (16c) of the first column (16).

7. System according to claim 6,
further comprising a discharge line (62) for removing high-boiling impurities from the lower section (16c) of the first column (16) to a withdrawal line (54).

8. Method for purifying recovered NMP from a lithium-ion battery production using a plant (10) according to any one of the preceding claims, comprising the steps:
- inputting the recovered NMP into the middle part of the first column (16);
- heating the first column (16) by means of the first evaporator (18) through the supply of thermal energy;
- extracting low-boiling impurities at the head (16b) of the first column (16);
- transferring pre-purified NMP from the lower part (16c) of the first column (16) or the cup draw-off (36b) into the lower part (38c) of the second column (38);
- evaporating the pre-purified NMP in the second evaporator (40) and passing it through the second column (38);
- extracting the vapour-phase NMP from the head (38b) of the second column (38) and compressing it in the compressor section (44) using the mechanical vapour compressor (46);
- inputting the compressed NMP into the inlet (40a) of the second evaporator (40), wherein the NMP is condensed in the second evaporator (40);
- extracting the condensed NMP at the outlet (40b) of the second evaporator (40); and
- extracting high-boiling impurities from the sump (38c) of the second column (38).

9. Method according to claim 8,
wherein in the first and second column (16, 38) a reduced pressure is maintained, particularly of less than 100 mbar.

10. Method according to any one of claims 8 and 9,
wherein the supply of thermal energy to the first evaporator (18) is carried out by means of steam or a heat transfer fluid.

11. Method according to any one of claims 8 to 10,
wherein a portion of the condensed NMP from the second evaporator (40) is returned as reflux into the second column (38).

12. Method according to any one of claims 8 to 11,
wherein the low-boiling impurities taken from the head (16b) of the first column (16) are condensed in the condenser (24) and preferably partially returned as reflux to the first column (16).

13. Method according to any one of claims 8 to 12,
wherein the recovered NMP contains less than 5% of low-boiling impurities; and/or wherein in the first column (16) the low-boiling impurities in the pre-purified NMP are reduced to less than 0.1%, preferably to less than 0.05%.

14. Method according to any one of claims 8 to 13,
wherein the high-boiling impurities withdrawn from the sump (38c) of the second column (38) are at least partially introduced into the lower part (16c) of the first column (16).

15. Method according to claim 14,
wherein the concentration of high-boiling impurities in the sump (38c) of the second column (38) during operation of the plant, relative to the initial concentration in the recovered NMP, is approximately 1 to 5, and the concentration of high-boiling impurities in the lower part (16c) of the first column (16) is increased compared to this value and ranges from greater than 1 to approximately 100.

## Revendications

1. Installation (10) de purification de NMP récupéré d'une production de batteries lithium-ion, comprenant :
- une première colonne (16) pour séparer des impuretés à bas point d'ébullition, qui présente dans sa partie centrale une alimentation (16a) pour le NMP récupéré et qui peut être chauffée par apport d'énergie thermique au moyen d'un premier évaporateur (18) ; et
- une deuxième colonne (38) pour séparer des impuretés à haut point d'ébullition,
- un condenseur (24) pour condenser les impuretés à bas point d'ébullition, qui est couplé à la tête (16b) de la première colonne (16),
dans laquelle
une conduite de raccordement (36) est prévue d'une partie inférieure (16c) de la première colonne (16) à une partie inférieure (38c) de la deuxième colonne (38) ; la deuxième colonne (38) peut être chauffée au moyen d'un deuxième évaporateur (40) ayant une entrée (40a) et une sortie (40b) pour le NMP purifié, et
une portion de compresseur (44) est associée à la deuxième colonne (38), laquelle s'étend d'une tête (38b) de la deuxième colonne (38) à l'entrée (40a) du deuxième évaporateur (40) et comprend un compresseur mécanique de vapeurs (46) pour l'introduction d'énergie dans les vapeurs de la deuxième colonne (38),
**caractérisée en ce qu'**une conduite de déplacement de gaz (56) est prévue entre la partie inférieure (16c) de la première colonne (16) et la partie inférieure (38c) de la deuxième colonne (38), et une conduite de purge d'air ayant une vanne de régulation (58) est prévue entre le deuxième évaporateur (40) et une portion centrale de la première colonne (16).

2. Installation (10) selon la revendication 1,
dans laquelle un échangeur de chaleur (14), en particulier un échangeur de chaleur à plaques, est placé en amont de l'alimentation (16a) de la première colonne (16), lequel est couplé d'autre part à la sortie (40b) du deuxième évaporateur (40).

3. Installation (10) selon l'une des revendications précédentes,
dans laquelle une pompe à vide (60) est associée au condenseur (24), laquelle est agencée pour maintenir une pression réduite dans l'installation.

4. Installation (10) selon l'une des revendications 1 à 3,
dans laquelle le premier et/ou le deuxième évaporateur (18, 40) est/sont réalisé(s) sous forme d'évaporateur à flot tombant ou d'évaporateur à circulation.

5. Installation selon l'une des revendications précédentes,
dans laquelle la première colonne (16) comprend un extracteur à godets (36b) à partir duquel s'étend la conduite de raccordement (36a) vers la partie inférieure (38c) de la deuxième colonne (38).

6. Installation selon la revendication 5,
comprenant en outre une recirculation de liquide (42b) agencée pour introduire au moins partiellement dans la partie inférieure (16c) de la première colonne (16) les impuretés à haut point d'ébullition prélevées dans le fond (38c) de la deuxième colonne (38).

7. Installation selon la revendication 6,
comprenant en outre une conduite d'évacuation (62) pour évacuer les impuretés à haut point d'ébullition de la partie inférieure (16c) de la première colonne (16) vers une conduite de prélèvement (54).

8. Procédé de purification de NMP récupéré d'une production de batteries lithium-ion au moyen d'une installation (10) selon l'une des revendications précédentes, comprenant les étapes consistant à :
- introduire le NMP récupéré dans la partie centrale de la première colonne (16) ;
- chauffer la première colonne (16) par apport d'énergie thermique au moyen du premier évaporateur (18);
- prélever les impuretés à bas point d'ébullition à la tête (16b) de la première colonne (16) ;
- transférer le NMP prépurifié de la partie inférieure (16c) de la première colonne (16) ou de l'extracteur à godets (36b) à la partie inférieure (38c) de la deuxième colonne (38) ;
- évaporer le NMP prépurifié dans le deuxième évaporateur (40) et le faire passer à travers la deuxième colonne (38) ;
- prélever le NMP sous forme de vapeur à la tête (38b) de la deuxième colonne (38) et le comprimer dans la portion de compresseur (44) au moyen du compresseur mécanique de vapeurs (46) ;
- introduire le NMP comprimé dans l'entrée (40a) du deuxième évaporateur (40), le NMP se condensant dans le deuxième évaporateur (40) ;
- prélever le NMP condensé à la sortie (40b) du deuxième évaporateur (40) ; et
- prélever les impuretés à haut point d'ébullition du fond (38c) de la deuxième colonne (38).

9. Procédé selon la revendication 8,
dans lequel une pression réduite, en particulier inférieure à 100 mbars, est maintenue dans les première et deuxième colonnes (16, 38).

10. Procédé selon l'une des revendications 8 et 9,
dans lequel l'apport d'énergie thermique au premier évaporateur (18) est réalisé au moyen de vapeur ou d'un fluide caloporteur.

11. Procédé selon l'une des revendications 8 à 10,
dans lequel une partie du NMP condensé provenant du deuxième évaporateur (40) est recyclée dans la deuxième colonne (38) sous forme de reflux.

12. Procédé selon l'une des revendications 8 à 11,
dans lequel les impuretés à bas point d'ébullition prélevées en tête (16b) de la première colonne (16) sont condensées dans le condenseur (24) et de préférence partiellement recyclées dans la première colonne (16) sous forme de reflux.

13. Procédé selon l'une des revendications 8 à 12,
dans lequel le NMP récupéré contient moins de 5 % d'impuretés à bas point d'ébullition ; et/ou
dans la première colonne (16), les impuretés à bas point d'ébullition dans le NMP prépurifié sont appauvries à moins de 0,1 %, de préférence à moins de 0,05 %.

14. Procédé selon l'une des revendications 8 à 13,
dans lequel les impuretés à haut point d'ébullition prélevées en fond (38c) de la deuxième colonne (38) sont introduites au moins partiellement dans la partie inférieure (16c) de la première colonne (16).

15. Procédé selon la revendication 14,
dans lequel la concentration des impuretés à haut point d'ébullition dans le fond (38c) de la deuxième colonne (38) pendant un fonctionnement de l'installation est d'environ 1 à 5 par rapport à la concentration initiale dans le NMP récupéré, et la concentration des impuretés à haut point d'ébullition dans la partie inférieure (16c) de la première colonne (16) est augmentée par rapport à cette valeur et se situe dans la plage allant de plus de 1 à environ 100.
